# EUROPEAN PATENT APPLICATION

(11) **EP 4 527 427 A1**
(43) Date of publication of application: **26.03.2025**
(21) Application number: 23306443.5
(22) Date of filing: 31.08.2023
(51) Int. Cl.: A61M 5/00

(54) **NEST WITH ENLARGED INTERIOR CHIMNEY CHAMFER**

(71) Applicant: Becton, Dickinson and Company, Franklin Lakes, NJ 07417-1880 (US)
(72) Inventor: LE LOC'H, Clémentine, 38240 Meylan (FR); PAULET, Mathilde, 38100 Grenoble (FR); CIBOULET, Antoine, 38000 Grenoble (FR)
(74) Representative: Germain Maureau

(57) **Abstract**

A nest for the storage of medical device components is provided. The nest includes a main wall having a top surface and a bottom surface and a plurality of chimneys for the storage of a plurality of medical device components therein. Each of the chimneys includes a sidewall extending downward from the bottom surface of the nest to define the chimney, with each of the chimneys having an upper opening and a lower opening. The sidewall of each of the chimneys has a top sidewall portion and a bottom sidewall portion, with a dividing point separating the top sidewall portion and the bottom sidewall portion. A diameter of the chimney at the dividing point is less than a diameter of the upper opening and less than a diameter of the lower opening.

## Description

### BACKGROUND

### Field of the Disclosure

The present disclosure relates generally to nest arrangements for the packaging of medical containers such as, e.g., syringes. More particularly, the present disclosure relates to nest arrangements having chimneys with enlarged interior chamfer configurations to compensate for possible curvature and/or deflection of the nest.

### Description of the Related Art

As is known in the art, medical containers (such as, e.g., syringes) for delivery or storage of a medicament, drug, or vaccine utilize a plunger stopper in contact with an inside surface of a generally tubular syringe barrel in order to draw a substance into (or expel a substance from) the device by way of a plunger rod.

Currently, many such devices are filled and/or assembled using automated filling machines. Not only do such machines improve productivity and accuracy, but they also provide for a substantially sterile and aseptic filling environment. The various components of the devices (e.g., plunger stoppers, syringe barrels, etc.) may be separately provided within the filling machines to enable at least some level of automated assembly.

Typically, a plurality of medical containers are packaged in nests having numerous "chimneys" formed therein to hold at least the syringe barrels, with each nest configured to be held within a substantially box-shaped tub when transported and/or introduced into the filling machine. Removal of the medical containers from this tub often includes peeling a sealing cover from the tub, removing the nest holding the medical containers from the tub, and then removing the medical containers from the nest by axially sliding the medical containers relative to the nest. Before being removed from the nest and packaged individually, the medical containers are filled with a pharmaceutical composition agent by means of a filling machine.

The nest is typically a plate-shaped tray that may be configured to support more than one hundred medical containers. Usually, the nest comprises numerous chimneys aligned according to predetermined rows, each chimney being configured to permit insertion of at least the barrel of one medical container. However, while nest capacity may be optimized by providing such numerous chimneys, the molding parameters needed to create such a nest are complex and require high pressures. As a consequence, unpredictable curvature and/or deflection of the nests are common after molding and/or after sterilization, even in nest designs incorporating reinforcing ribs.

In some instances, nest curvature and/or deflection may be significant enough to adversely affect performance of the automated filling machines. For example, syringe barrels may become lodged within associated chimneys during insertion and/or extraction from the nest, as the curvature or deflection of the nest (and, thus, associated angulation of the chimney) may not allow for a typical vertical placement or removal of the medical containers. Consequently, the automated processing lines may need to be stopped until the medical container(s) are able to be dislodged from the nest, leading to undesirable downtime and maintenance costs, as well as potential damage to the medical container(s).

### SUMMARY

In view of the foregoing, there exists a need for a nest designed specifically to account for potential curvature and/or deflection due to manufacturing processes and/or sterilization, thereby enabling consistent insertion and/or extraction of medical containers therefrom.

Embodiments of the present disclosure are directed to a nest for the storage of medical device components. The nest includes a main wall having a top surface and a bottom surface and a plurality of chimneys for the storage of a plurality of medical device components therein. Each of the chimneys includes a sidewall extending downward from the bottom surface of the nest to define the chimney, with each of the chimneys having an upper opening and a lower opening. The sidewall of each of the chimneys has a top sidewall portion and a bottom sidewall portion, with a dividing point separating the top sidewall portion and the bottom sidewall portion. A diameter of the chimney at the dividing point is less than a diameter of the upper opening and less than a diameter of the lower opening.

In some embodiments, the top sidewall portion is angled outwardly from the dividing point to the upper opening and the bottom sidewall portion is angled outwardly from the dividing point to the lower opening, such that a bottom portion of the chimney has a frustoconical shape and a top portion of the chimney has an inverted frustoconical shape.

In some embodiments, a draft angle of each of the top sidewall portion and the bottom sidewall portion is between 0° and 5°.

In some embodiments, a length of the top sidewall portion is greater than a length of the bottom sidewall portion.

In some embodiments, each of the plurality of chimneys includes a chamfered surface between the top surface and the top sidewall portion.

In some embodiments, with the top surface angled at greater than 0°, the medical device component, when inserted, makes contact with an inner edge of the chamfered surface and with the top sidewall portion along a first side surface of the medical device component and makes contact with the bottom sidewall portion along a second side surface of the medical device component, in order to store the medical device component in a generally vertical orientation.

In some embodiments, the medical device component is insertable in and extractable from the chimney with the top surface angled between 0° and 5°.

In some embodiments, the diameter of the upper opening is larger than the diameter of the lower opening.

In some embodiments, the dividing point is a horizontally oriented ledge extending inwardly from a bottom edge of the top sidewall portion to a top edge of the bottom sidewall portion, and wherein the top sidewall portion is oriented generally vertically between the upper opening and an outer edge of the ledge and the bottom sidewall portion is angled inwardly from the lower opening to an inner edge of the ledge.

In some embodiments, wherein a draft angle of the top sidewall portion is between 0 and 5° degrees and a draft angle of the bottom sidewall portion is between 0° and 5°.

In some embodiments, the diameter of the upper opening is larger than the diameter of the lower opening.

In some embodiments, a length of the top sidewall portion is greater than a length of the bottom sidewall portion.

In some embodiments, the medical device component is insertable in and extractable from the chimney with the top surface angled between 0° and 5°.

In some embodiments, the diameter of the upper opening is approximately 17.6 mm, the diameter of the lower opening is approximately 16.3 mm, and the diameter of the chimney at the dividing point is approximately 15.5 mm.

In some embodiments, the medical device components stored in the nest are syringe barrels.

Further details and advantages of the present disclosure will be understood from the following detailed description read in conjunction with the accompanying drawings.

### BRIEF DESCRIPTION OF THE DRAWINGS

FIG. 1 is a cross-sectional view of a chimney included in a nest for storing medical devices, as known in the prior art;
FIG. 2A is a top perspective view of a nest for storing medical devices, in accordance with an embodiment of the present disclosure;
FIG. 2B is a bottom perspective view of the nest of FIG. 2A;
FIG. 3 is a side perspective view of the nest of FIG. 2A, showing a curvature or deflection that may occur during manufacturing thereof;
FIG. 4 is a cross-sectional view of a chimney included in the nest of FIG. 2A, taken along line a-a, in accordance with an embodiment of the present disclosure;
FIG. 5 illustrates a medical device inserted into the chimney of FIG. 4 when a top surface of the nest is deflected; and
FIG. 6 is a cross-sectional view of a chimney included in the nest of FIG. 2A, taken along line a-a, in accordance with another embodiment of the present disclosure.

### DESCRIPTION OF THE INVENTION

The following description is provided to enable those skilled in the art to make and use the described aspects contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For the purposes of the description hereinafter, the terms "upper", "lower", "right", "left", "vertical", "horizontal", "top", "bottom", "lateral", "longitudinal", and derivatives thereof shall relate to the invention as it is oriented in the drawings. However, it is to be understood that the invention may assume various alternative variations, except where expressly specified to the contrary. It is also to be understood that the specific devices illustrated in the attached drawings, and described in the following specification, are simply exemplary aspects of the invention. Hence, specific dimensions and other physical characteristics related to the aspects disclosed herein are not to be considered as limiting.

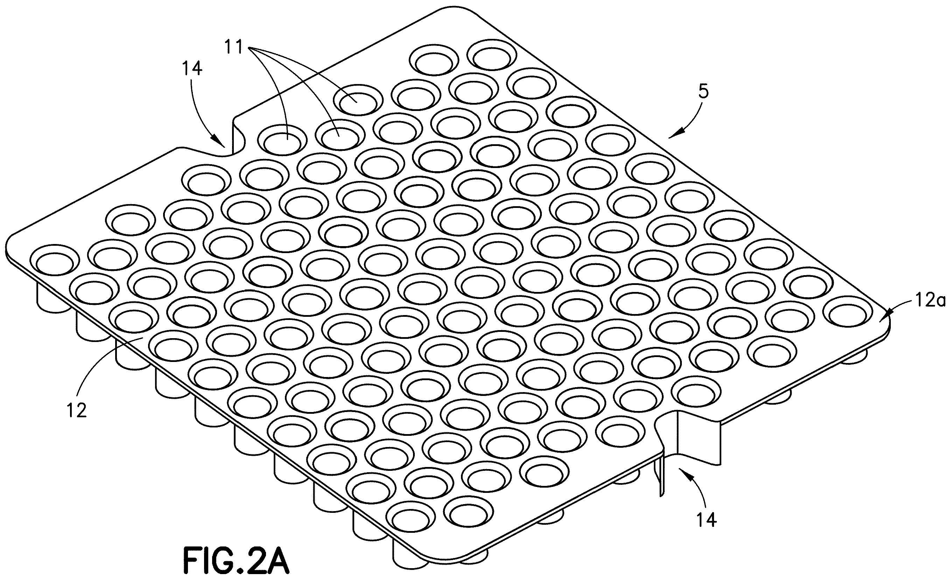

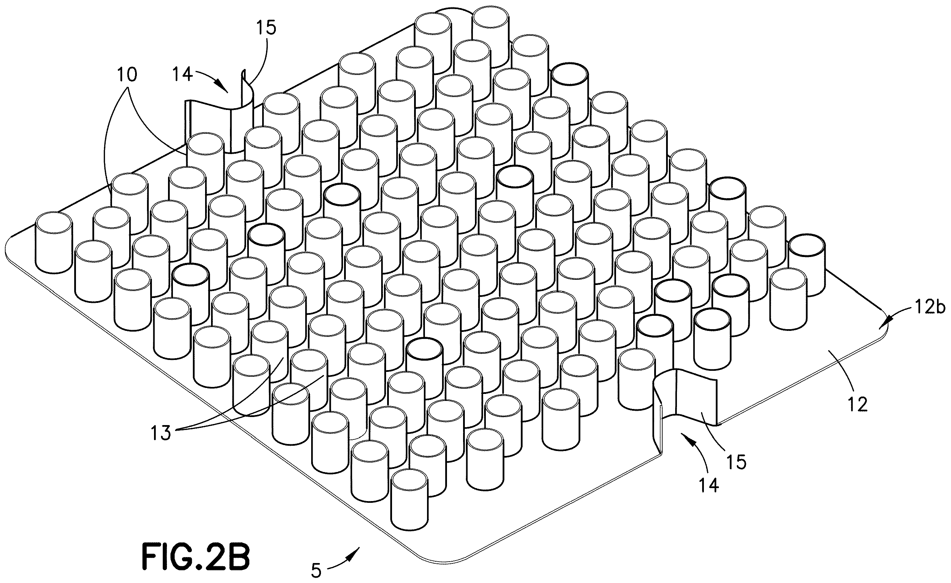

Referring to FIGS. 2A and 2B, a nest 5 in accordance with one aspect of the present disclosure is shown. The nest 5 is configured to store a plurality of medical devices (not shown) therein in a way that provides for desired positioning and alignment of the medical devices to allow for accurate handling by the robotic components of the automated filling machines. In some embodiments, and as referenced hereafter, the medical devices stored in the nest 5 are syringe barrels, but it is to be understood that the nests described in the present disclosure are not limited to use with syringe barrels, and may be utilized with other elements and/or devices. While not shown, a plurality of nests 5 are capable of being removably retained within a tub or other holding arrangement, with the nests 5 configured to be stackable vertically atop one another.

According to embodiments, the nest 5 is formed as a single part and, more specifically, is formed of a molded plastic material. The nest 5 has a substantially square or rectangular shape that matches that of a tub within which it may be retained. The nest 5 includes a plurality of receptacles or "chimneys" 10 formed therein, each of which is capable of holding a syringe barrel therein for access and removal by components of an autonomous filling machine. In some embodiments, the chimneys 10 are sized and configured so as to allow for the syringe barrels to pass therethrough until flanges of the syringe barrels abut the upper ends of the chimneys 10. FIGS. 2A and 2B show nest 5 with a "top" surface thereof being visible (FIG. 2A) and a "bottom" surface thereof being visible (FIG. 2B), with the orientation of nest 5 shown in FIG. 2B providing for insertion of syringe barrels into chimneys 10 from the top side of nest 5 and down through chimneys 10. Upon insertion/securing of syringe barrels within the chimneys, the nest 5 would be oriented so that chimneys 10 face up, with the nest 5 placed within a tub (not shown), such that the nest 5 rests on a ledge of the tub with the syringe barrels extending down through chimneys 10.

Each chimney 10 includes an upper opening 11 formed in a top surface 12a of a flat main wall 12 of the nest 5, while the chimney 10 is formed by a sidewall 20 that extends down from a bottom surface 12b of the main wall 12 of the nest 5. The diameter and size of each chimney 10 may vary based on the type of syringe barrel utilized during a particular filling operation. In some embodiments, the nest 5 may be configured to retain, e.g., 100 syringe barrels sized for use in a 1-3 mL syringe or 64 syringe barrels sized for use in a 5 mL syringe. However, it is to be understood that the nest 5 may be configured to hold more or fewer syringe barrels, as well as syringe barrels of differing size(s).

In some embodiments, the nest 5 may include median finger notches 14 on two opposed edges of the main wall 12, with notch walls 15 delimiting the finger notches 14. The finger notches 14 allow the insertion of a user's fingers therethrough gripping the nest 5, and the notch walls 15 provide increased contact surfaces for these fingers. However, it is to be understood that the nest 5 may be provided without the finger notches 14 and/or notch walls 15.

While not shown in FIGS. 2A and 2B, it is recognized that the nest 5 may include additional features that allow for alignment and insertion of the nest into a storage tub. For example, the nest 5 may include lateral positioning pillar notches on the first and second opposed edges that allow the insertion of the nest 5 on supporting pillars of a tub. In some embodiments, the nest 5 may also include features that aid in rigidifying the nest 5. While not shown in FIGS. 2A and 2B, it is recognized that nest 5 could include a plurality of radial connection walls that extend between adjacent chimneys 10 in order to effectively connect the sidewalls 20 of chimneys 10 to one another, thereby aiding in rigidifying the nest 5.

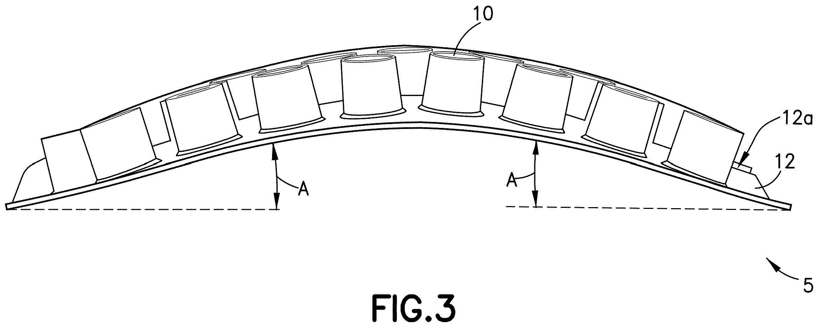

Even in instances where radial connection walls 13 and/or other rigidifying features are provided, the complex molding parameters needed to create the nest 5 may lead to unpredictable curvature and/or deflection of the nest 5 after molding and/or sterilization, such as shown in FIG. 3 where top surface 12a of main wall 12 has a deflection angle A. Accordingly, as will be described in further detail below, the chimneys 10 in accordance with an aspect of the present disclosure are formed so as to accommodate curvature and/or deflection of the nest 5, thereby allowing the cylindrical bodies of syringe barrels to be inserted into and/or extracted from the chimneys 10 with a reduced likelihood of binding within the chimneys 10.

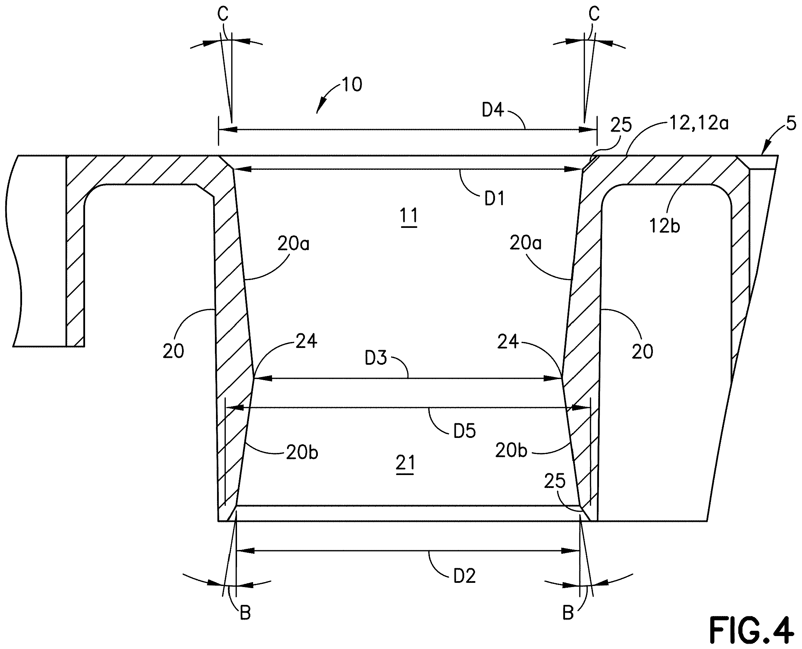

Specifically, referring to FIG. 4, a cross-sectional view of a single chimney 10 of the nest 5 in accordance with an aspect of the present disclosure is illustrated. It is to be understood that each chimney 10 of the plurality of chimneys 10 of nest 5 may be configured as is shown in FIG. 3. Alternatively, in accordance with other embodiments, only a subset of chimneys 10 may be configured as is shown in FIG. 3.

As shown in FIG. 3, the chimney 10 includes upper opening 11 and a lower opening 21 at opposing ends thereof. Each of the upper opening 11 and lower opening 21 is coaxially surrounded by the chimney 10, such that the cylindrical body of a syringe barrel may be inserted to and/or extracted from chimney 10 via the upper and lower openings 11, 21.

The chimney 10 is generally defined by the sidewall 20 that extends down from the bottom surface 12b of main wall 12, between the upper opening 11 and the lower opening 21. The sidewall 20 may be characterized as having a top sidewall portion 20a and a bottom sidewall portion 20b, with the top sidewall portion 20a positioned adjacent upper opening 11 and extending down from main wall 12 and the bottom sidewall portion 20b positioned beneath the top sidewall portion 20a, distal from main wall 12. The top sidewall portion 20a and bottom sidewall portion 20b are separated by a dividing point 24 that, according to embodiments, may be a structurally defined dividing point 24 between the top and bottom sidewall portions 20a, 20b or a structurally undefined dividing point 24. In the illustrated embodiment, the dividing point 24 is a edge/ridge junction from which the top sidewall portion 20a and bottom sidewall portion 20b diverge out at differing angles.

As shown in FIG. 3, each of the top sidewall portion 20a and bottom sidewall portion 20b has a draft angle, i.e., an angle of inclination of the sidewall portion, with the top sidewall portion 20a having a draft angle C between the upper opening 11 and the dividing point 24 and the bottom sidewall portion 20b having a draft angle B between the lower opening 21 and the dividing point 24. That is, the top sidewall portion 20a is angled outwardly from the dividing point 24 to the upper opening 11 at a first draft angle C and the bottom sidewall portion 20b is angled outwardly from the dividing point 24 to the lower opening 21 at a second draft angle B, which may be the same or different from draft angle C. Accordingly, a bottom portion of the chimney 10 thus has a frustoconical shape, while a top portion of the chimney 10 has an inverted frustoconical shape. In some embodiments, the draft angle C, B of the top sidewall portion 20a and bottom sidewall portion 20b of chimney is relatively small. For example, the draft angle C, B of the top and bottom sidewall portions 20a, 20b may be between 0° and 5°.

According to embodiments of the disclosure, the dividing point 24 between the top and bottom sidewall portions 20a, 20b may be vertically centered between the upper and lower openings 11, 21 or, as shown in FIG. 4, may be vertically offset between the upper and lower openings 11, 21. Also, according to embodiments of the disclosure, the draft angle C of the top sidewall portion 20a may be equal and opposite to the draft angle B of the bottom sidewall portion 20b or may be different from the draft angle B of the bottom sidewall portion 20b. Based on the length and the draft angles C, B of the top and bottom sidewall portions 20a, 20b, the size of the upper opening 11 and the size of the lower opening 21, i.e., a diameter D1 of the upper opening 11 and a diameter D2 of the lower opening 21, may be the same or different. In the illustrated embodiment, the top sidewall portion 20a is longer than the bottom sidewall portion 20b, while the draft angle of the bottom sidewall portion 20b is greater than the draft angle of the top sidewall portion 20a, with the sidewall portion lengths and draft angles resulting in the diameter D1 of the upper opening 11 and the diameter D2 of the lower opening 21 being approximately equal.

With the top and bottom sidewall portions 20a, 20b that define chimney 10 being structured and arranged as described above, the chimney 10 is internally formed such that its smallest internal diameter D3, at the dividing point 24, is less than the diameter D1 of the upper opening 11 and less than the diameter D2 of the lower opening 21. The diameter D3 of the chimney 10 at the dividing point 24 is determined based on the diameter of the syringe barrel to be received in the chimney 10, with the diameter D3 being approximately equal to that of the syringe barrel so as to secure the syringe barrel within the chimney 10 when stored therein. For a syringe barrel of 5 ml in volume, for example, the diameter D3 may thus be approximately 15.5 mm (+/- 0.1 mm). As the diameters D1, D2 of the upper opening 11 and lower opening 21 are larger than the diameter D3 of the chimney 10 at dividing point 24, the diameters D1, D2 of the upper opening 11 and lower opening 21 may thus be approximately 17.6 mm and 16.3 mm (+/- 0.1 mm), respectively, for a syringe barrel of 5 ml in volume.

According to some embodiments of the disclosure, the chimney 10 may also include a chamfered edge 25 at both the top and bottom thereof - i.e., between the main wall 12 and the top sidewall portion 20a and on bottom sidewall portion 20b adjacent opening 21. These chamfered edges 25 may have an outside diameter D4, D5 larger than the diameters D1, D2 of the upper and lower openings 11, 21, thereby providing for easier insertion/removal of a syringe barrel into and out from the chimney 10. The combination of chamfered edges 25 and the draft angles C, B of the top and bottom sidewall portions 20a, 20b provides a chimney 10 having what may be termed as "enlarged interior chamfer configurations" that aid in insertion and/or extraction of a syringe barrel from the nest 5, as explained in more detail below.

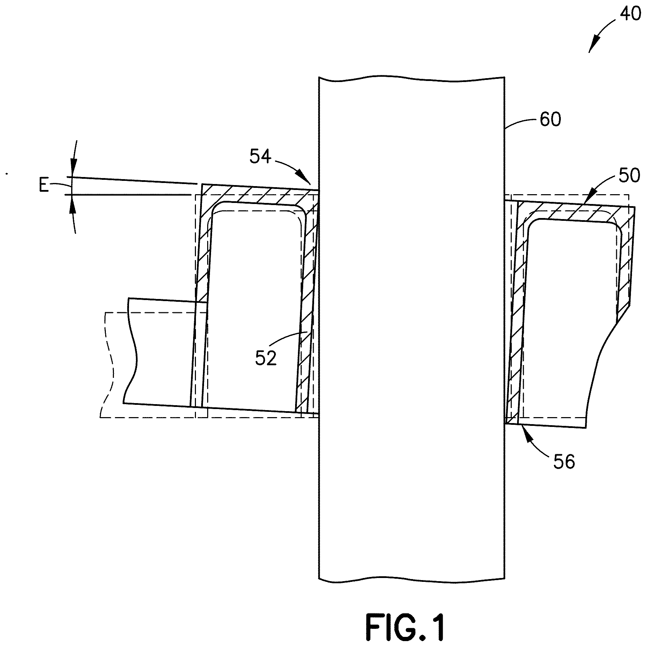

The structure of the chimney 10 as described above beneficially allows for some variance to be present in the nest 5 (resulting from the molding fabrication thereof) without affecting the performance thereof. That is, as described above and shown in FIG. 1, in a typical nest 40, a curvature and/or deflection of the nest 40 may occur during fabrication thereof that adversely affects performance of an automated filling machine, such as when the curvature or deflection of the top surface 50 of the nest 40 (and, thus, an associated angulation of the chimney 54) does not allow for a typical vertical placement or removal of a syringe barrel 60. In existing nests 40, a curvature or deflection E of the top surface 50 of more than 2.5 mm, for example, may result in syringe barrels 60 becoming lodged within associated chimneys 54 during insertion and/or extraction of a syringe barrel 60 from the nest 40.

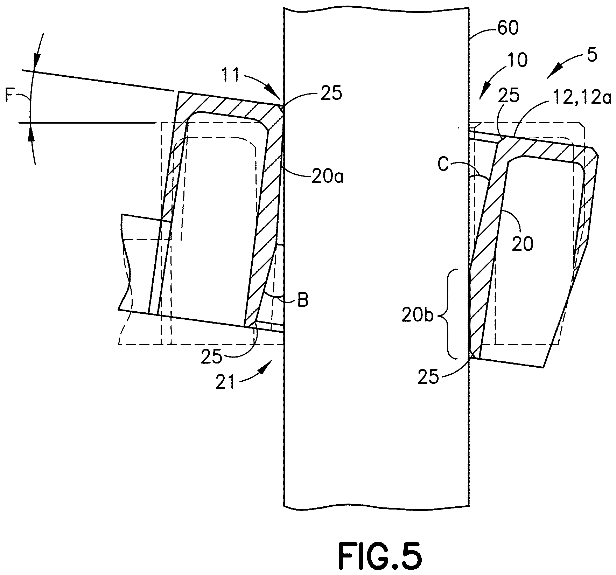

As shown best in FIG. 5, where a syringe barrel is shown inserted into a chimney 10 of nest 5, the structure of chimney 10 allows for a curvature or deflection of the top surface 12a of nest 5 to be present without affecting the vertical placement or removal of a syringe barrel 60 from the chimney 10. In the illustrated embodiment, a curvature or deflection of the top surface 12a of up to 5° (i.e., between 0° and 5°) may be present without affecting the ability of a syringe barrel 60 to be inserted to or extracted from the chimney 10. As can be seen in FIG. 5, when the top surface 12a is oriented at an angle F and a syringe barrel 60 is inserted in chimney 10, the syringe barrel 60 makes contact with an inner edge of the chamfered surface 25 and with the top sidewall portion 20a along one side of the syringe barrel 60 and makes contact with the bottom sidewall portion 20b along the opposite side of the syringe barrel 60. The arrangement of the syringe barrel 60 relative to the chimney 10 in this manner, facilitated by the size of the upper and lower openings 11, 21 and the orientation of the top and bottom sidewall portions 20a, 20b, allows for the syringe barrel 60 to be stored in a generally vertical orientation. The generally vertical orientation of the syringe barrel 60 within chimney 10 enables a smooth insertion and extraction of the syringe barrel 60 from the nest 5, thereby preventing occurrences of the syringe barrel 60 becoming lodged within a respective chimney 10.

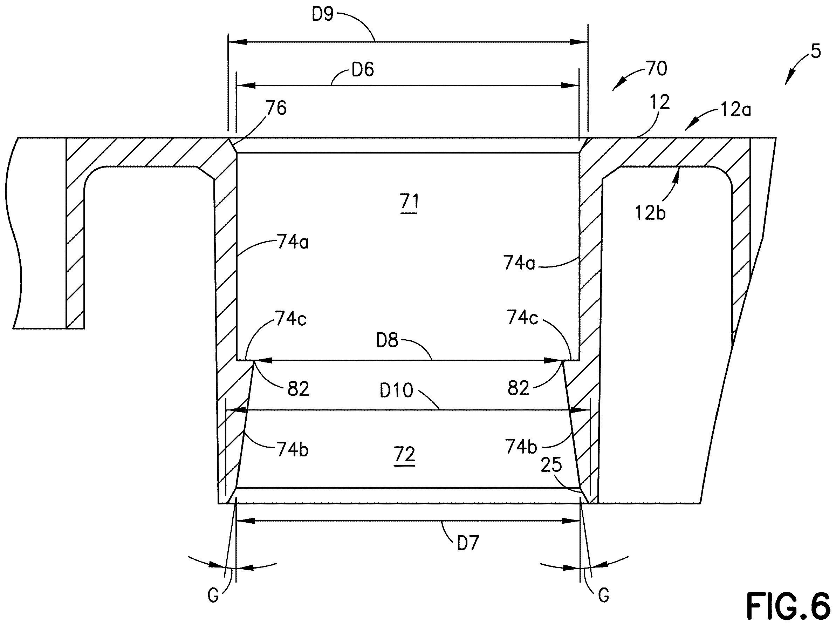

Referring now to FIG. 6, a cross-sectional view of a single chimney 70 of the nest 5 is illustrated in accordance with another aspect of the present disclosure. It is to be understood that each chimney 70 of the plurality of chimneys 70 of nest 5 may be configured as is shown in FIG. 5 or, alternatively, only a subset of chimneys 70 may be configured as is shown in FIG. 5.

As shown in FIG. 6, the chimney 70 includes an upper opening 71 and a lower opening 72 at opposing ends thereof. Each of the upper opening 71 and lower opening 72 is coaxially surrounded by the chimney 70, such that the cylindrical body of a syringe barrel may be inserted to and/or extracted from chimney 70 via the upper and lower openings 71, 72.

The chimney 70 is generally defined by a sidewall 74 that extends down from main wall 12, between the upper opening 71 and the lower opening 72. The sidewall 74 may be characterized as having a top sidewall portion 74a and a bottom sidewall portion 74b, with the top sidewall portion 74a positioned adjacent upper opening 71 and extending down from main wall 12 and the bottom sidewall portion 74b positioned beneath the top sidewall portion 74a, distal from main wall 12. The top sidewall portion 74a and bottom sidewall portion 74b are separated by a dividing point 82 that, in the illustrated embodiment, is a horizontally oriented ledge 74c that separates the top sidewall portion 74a from the bottom sidewall portion 74b. The horizontally oriented ledge 74c extends inwardly from a bottom edge of the top sidewall portion 74a to a top edge of the bottom sidewall portion 74b.

As shown in FIG. 6, the top sidewall portion 74a has a generally vertical orientation while the bottom sidewall portion 74b has a draft angle G between the lower opening 72 and the dividing point 82, with the bottom sidewall portion 74b being angled outwardly from the ledge 74c to the lower opening 72. According to embodiments, the draft angle G of bottom sidewall portion 74b may be between 0° and 5°.

According to embodiments of the disclosure, the dividing point 82 between the top and bottom sidewall portions 74a, 74b may be vertically centered between the upper and lower openings 71, 72 or, as shown in FIG. 6, may be vertically offset between the upper and lower openings 71, 72. Based on the length of the top and bottom sidewall portions 74a, 74b and the draft angle G of the bottom sidewall portion 74b, the size of the upper opening 71 and the size of the lower opening 72, i.e., a diameter D6 of the upper opening 71 and a diameter D7 of the lower opening 72, may be the same or different. In the illustrated embodiment, the top sidewall portion 74a is longer than the bottom sidewall portion 74b and the draft angle G of the bottom sidewall portion 74b is such that the diameter D6 of the upper opening 71 and the diameter D7 of the lower opening 72 are approximately equal.

With the top and bottom sidewall portions 74a, 74b that define chimney 70 being structured and arranged as described above, the chimney 70 is internally formed such that its smallest internal diameter D8, at the dividing point 82, is less than the diameter D6 of the upper opening 71 and less than the diameter D7 of the lower opening 72. As previously described regarding the chimney 10 of FIG. 4, the diameter D8 of the chimney 70 at the dividing point 82 is determined based on the diameter of the syringe barrel to be received in the chimney 70, with the diameter D8 being approximately equal to that of the syringe barrel so as to secure the syringe barrel within the chimney 70 when stored therein. As an example, the diameter D8 may thus be approximately 15.5 mm, while the diameters D6, D7 of the upper opening 71 and lower opening 72 may thus be approximately 17.6 mm and 16.3 mm, respectively.

According to some embodiments of the disclosure, the chimney 70 may also include chamfered edges 76 at both the top and bottom thereof - i.e., between the main wall 12 and the top sidewall portion 74a and on bottom sidewall portion 74b adjacent opening 72. These chamfered edges 76 may have an outside diameter D9, D10 larger than the diameters D6, D7 of the upper and lower openings 71, 72, thereby providing for easier insertion of a syringe barrel into the chimney 70 and removal of the syringe barrel from the chimney 70.

As described above regarding the embodiment of FIG. 4, the structure of the chimney 70 beneficially allows for a curvature or deflection of the top surface 12a of nest 5 to be present without affecting the vertical placement or removal of a syringe barrel from the chimney 70. In the illustrated embodiment, a curvature or deflection of the top surface 12a of up to 5° (i.e., between 0° and 5°) may be present without affecting the ability of a syringe barrel to be inserted to or extracted from the chimney 70 in a generally vertical orientation, so as to prevent occurrences of the syringe barrel becoming lodged within a respective chimney 70. Additionally, the inclusion of the ledge 74c in the sidewall 74 chimney 70 further increases and optimizes a thickness of the sidewall of chimney 70, so as to improve molding quality in the nest 5 that reduces sink mark risks.

Embodiments of the disclosure thus provide a nest having chimneys therein that counteract a high curvature of a top surface of the nest that might result from molding and/or sterilization of the nest. A nest with a chimney structure as described in accordance with embodiments of the present disclosure provides a higher flatness tolerance window for the top surface of the nest within which the nest may be molded and sterilized, while still allowing syringe barrels to be stored in a generally vertical orientation in the nest, such that the syringe barrels will not become lodged within the chimneys when they are inserted into or extracted out therefrom. Accordingly, the nests of the present disclosure can minimize the downtime of automated processing lines (i.e., automated filling machines) with which the nests are used.

While several embodiments of nests and nest arrangements are shown in the accompanying figures and described hereinabove in detail, other embodiments will be apparent to, and readily made by, those skilled in the art without departing from the scope and spirit of the invention. For example, it is to be understood that this disclosure contemplates, to the extent possible, that one or more features of any embodiment can be combined with one or more features of any other embodiment. Accordingly, the foregoing description is intended to be illustrative rather than restrictive.

## Claims

1. A nest for the storage of medical device components, comprising:
a main wall comprising a top surface and a bottom surface; and
a plurality of chimneys for the storage of a plurality of medical device components therein, wherein each of the plurality of chimneys comprises a sidewall extending downward from the bottom surface of the main wall to define the chimney, and wherein each of the plurality of chimneys comprises an upper opening and a lower opening;
**characterized in that** the sidewall of each of the plurality of chimneys comprises a top sidewall portion and a bottom sidewall portion, with a dividing point separating the top sidewall portion and the bottom sidewall portion, wherein a diameter of the chimney at the dividing point is less than a diameter of the upper opening and less than a diameter of the lower opening.

2. The nest of claim 1, wherein the top sidewall portion is angled outwardly from the dividing point to the upper opening and the bottom sidewall portion is angled outwardly from the dividing point to the lower opening, such that a bottom portion of the chimney has a frustoconical shape and a top portion of the chimney has an inverted frustoconical shape.

3. The nest of claim 2, wherein a draft angle of each of the top sidewall portion and the bottom sidewall portion is between 0° and 5°.

4. The nest of claim 2 or claim 3, wherein a length of the top sidewall portion is greater than a length of the bottom sidewall portion.

5. The nest of any of claims 2-4, wherein each of the plurality of chimneys comprises a chamfered surface between the top surface and the top sidewall portion.

6. The nest of claim 5, wherein with the top surface angled at greater than 0°, a medical device component, when inserted, makes contact with an inner edge of the chamfered surface and with the top sidewall portion along a first side surface of the medical device component and makes contact with the bottom sidewall portion along a second side surface of the medical device component, in order to store the medical device component in a generally vertical orientation.

7. The nest of any of claims 1-6, wherein a medical device component is insertable in and extractable from the chimney with the top surface angled between 0° and 5°.

8. The nest of any of claims 1-7, wherein the diameter of the upper opening is larger than the diameter of the lower opening.

9. The nest of claim 1, wherein the dividing point comprises a horizontally oriented ledge extending inwardly from a bottom edge of the top sidewall portion to a top edge of the bottom sidewall portion, and wherein the top sidewall portion is oriented generally vertically between the upper opening and an outer edge of the ledge and the bottom sidewall portion is angled inwardly from the lower opening to an inner edge of the ledge.

10. The nest of claim 9, wherein a draft angle of the top sidewall portion is between 0 and 5° degrees and a draft angle of the bottom sidewall portion is between 0° and 5°.

11. The nest of claim 10, wherein the diameter of the upper opening is larger than the diameter of the lower opening.

12. The nest of any of claims 9-11, wherein a length of the top sidewall portion is greater than a length of the bottom sidewall portion.

13. The nest of claim 12, wherein a medical device component is insertable in and extractable from the chimney with the top surface angled between 0° and 5°.

14. The nest of any of claims 1-13, wherein the diameter of the upper opening is approximately 17.6 mm, the diameter of the lower opening is approximately 16.3 mm, and the diameter of the chimney at the dividing point is approximately 15.5 mm.

15. The nest of any of claims 1-14, wherein the medical device components comprise syringe barrels.
